# EUROPEAN PATENT SPECIFICATION

(11) **EP 0 620 437 B1**
(45) Date of publication and mention of the grant of the patent: **24.07.2002**
(21) Application number: 94105740.8
(22) Date of filing: 14.04.1994
(51) Int. Cl.: G01N 33/52, C12Q 1/54, C12Q 1/28

(54) **Multilayer test strip**
Mehrschichtiger Testträger
Dispositif multicouche

(30) Priority: 15.04.1993 US 48657
(43) Date of publication of application: 19.10.1994
(73) Proprietor: DIAGNOSTIC SOLUTIONS, INC., Irvine, CA 92718 (US)
(72) Inventor: Krantz, Gary, Laguna Beach CA 92651 (US); Chen, Shuenn-Tzong, Irvine CA 92720 (US); Zipp, Adam, Laguna Hills CA 92653 (US); Zeng, Joanne, Santa Ana CA 92705 (US)
(74) Representative: Munk, Ludwig, Dipl.-Ing.

(56) References cited:
- EP-A- 0 103 901
- EP-A- 0 162 301
- EP-A- 0 162 302
- EP-A- 0 239 990
- EP-A- 0 256 806
- EP-A- 0 322 882
- EP-A- 0 325 398
- EP-A- 0 416 588
- EP-A- 0 418 169

## Description

### BACKGROUND OF THE INVENTION

This invention relates to a test strip for use in testing liquids, and, more particularly, to a multilayer test strip of particular value in optical reflectance testing for the amount of glucose in blood by contacting a sample of the blood to the test strip.

A reagent-containing test strip provides a convenient and easy approach for conducting certain types of chemical analysis. The test strip is normally prepared. by impregnating a porous material with a solution containing the reagent, and optionally other ingredients. This step is readily accomplished by dipping the porous material into the reagent solution. The test strip and solution are dried to fix the reagent in the porous matrix. Additional reagents may be added by impregnating the porous material with additional solutions containing the additional reagents and drying the porous material after each further treatment.

To conduct a test using the test strip, a liquid that may contain an active species is contacted with the test strip. The liquid dissolves reagents that were previously introduced into the porous material, so that these reagents can mix with each other and with the active species, if present. The subsequent reaction is selected to cause some measurable change in the test strip. For example, if one of the reagents is a dye, the test strip can be made to change color responsive to the presence of the reactive species.

A test strip may be used in several ways. One common approach is to dip the test strip into a sample of the liquid to be tested, and to thereafter observe the behavior of the test strip. In another approach, a droplet of the liquid is placed onto the test strip. The test strip is observed either with the eye or with an instrument structured and calibrated to make accurate measurements of the test strip.

In one such approach, described in US Patent 4,935,346, a test strip is affixed to an inert backing that has an aperture therethrough to the test strip. A droplet of a liquid to be analyzed, preferably blood in the case of the '346 patent, is placed in the aperture through the backing. The blood penetrates into a first side of the test strip, reacting with reagents in the test strip. The reagents include a dye that colors responsive to the presence of a particular active species in the liquid.

As this test is performed, the test strip and the backing are held in a measurement instrument that directs a light beam against an opposite, second side of the test strip. The light reflected from the second side is measured. Changes in the reflected light quantitatively indicate the presence of the active species in the fluid. The values obtained by this approach are usually not quite as accurate as those obtained by conventional wet chemical analysis. The test strip approach has the important advantage, however, that it can be used by a relatively unskilled person to obtain a close approximation of the amount of the active species present in the fluid sample, with the testing performed in a home or other location away from the chemical laboratory.

This testing approach has been developed into a successful commercial product. However, several shortcomings in the test strips themselves and the methodology of the testing have been observed. These shortcomings can result in inaccuracies in the measured final results. One problem is that the test strips have a relatively short use life. The test strips are normally packed in a hermetically sealed container with a desiccant to keep them dry. The shelf life of the test strips at ambient temperature is about 2 years before the container is first opened, but the use life tends to be only about 4 months after the container is opened. Since the test strips are often purchased by persons who attempt to use them after their use life has expired, it is particularly important to extend the use life as much as possible.

Another problem is that the test results tend to vary with the amount and properties of blood that is placed onto the aperture in the backing. Although the instructions may call for a "drop" of blood to be used, a "drop" is not quantitative. Inexperienced persons may supply droplets of varying sizes. The droplets can vary both because of variations in technique and also because the viscosity of the blood of different persons can vary over a considerable range. The commercially available test strips that are used in the approach of the '346 patent have been observed by the present inventors to produce varying results as a function of the size of the "drop" of blood that is used and the viscosity of the blood.

EP 0 416 588 A2 describes a conventional test instrument, which shows a reagent layer, a spreading layer and a cover with an opening, wherein according to the common practice with respect to such conventional test instruments the chemicals for glucose analysis of blood are placed into a single layer. This test instrument can be used as a measuring device for measuring blood-sugar values by measuring coloration of the reagent layer.

EP 0 162 302 A1 is directed to an integral multilayer analytical element utilizable for determination of an analyte in a liquid sample such as a body fluid. This document discloses in a very general manner that in the case that two or more reagents participate in the reaction these reagents can be incorporated into one or plural layers.

EP 0 162 301 A1 describes an integral multilayer analytical element utilizable for determination of an analyte in a liquid sample such as a body fluid. This multilayer analytical element comprises a porous spreading layer of woven vabric or knitted fabric, a water-absorbing layer containing a hydrophilic polymer and a light-transmissive water-impermeable support.

There is a need for an improved test strip to be used in reflectance analysis procedures. The present invention fulfills this need, and further provides related advantages.

### SUMMARY OF THE INVENTION

The present invention provides a test strip which has greater stability after opening than test strips now used in reflected light blood analytical techniques. The test strips of the invention also exhibit less dependence of the test results on the volume and viscosity of the blood sample than found in conventional test strips. The present test strips can otherwise be substituted for the conventional test strips.

In accordance with the invention, a multilayer test strip comprises a first layer having an indicator reagent impregnated into a porous substrate. A second layer having a reactive reagent overlies the first layer. A third layer, comprising a spreading agent that promotes the spreading of a liquid upon the third layer, overlies the second layer. An inert backing overlies the third layer and has an aperture therethrough to the third layer.

The test strip of the invention has two innovations that represent important advances over the prior test strip. First, the indicator reagent and the reactive reagent are placed into two separate layers, so that they cannot chemically interact during storage prior to use. Such chemical interaction is a primary cause of reduced shelf life and degradation of the operability of the test strip during storage.

Second, a spreading layer is provided to improve the spreading of the liquid sample droplet over the upper layer after it is placed in the aperture of the inert backing. The inventors observed that the variability in the coverage of the aperture with different droplet sizes and liquid properties is a primary cause of the variability in the test results using the prior test strips in reflectance testing. In the present approach, the droplet spreads uniformly over the entire surface of the layer exposed through the aperture in the backing, regardless of the droplet size. This spreading of the droplet and the resulting full, uniform coverage of the aperture contributes to a test result that is more nearly independent of the size and flow properties of the sample droplet.

The present approach provides a significant improvement in dry test strips of the type that is particularly useful in reflectance testing for the presence of active chemical species. Other features and advantages of the present invention will be apparent from the following more detailed description of the preferred embodiment, taken in conjunction with the accompanying drawings, which illustrate, by way of example, the principles of the invention.

### BRIEF DESCRIPTION OF THE DRAWINGS

Figure 1 is an elevational view of a first embodiment of the test strip of the invention;
Figure 2 is a perspective view of the test strip of the invention;
Figure 3 is an elevational view of a prior art test strip; and
Figure 4 is a process flow diagram for the preparation of the test strip of the invention.

### DETAILED DESCRIPTION OF THE PREFERRED EMBODIMENTS

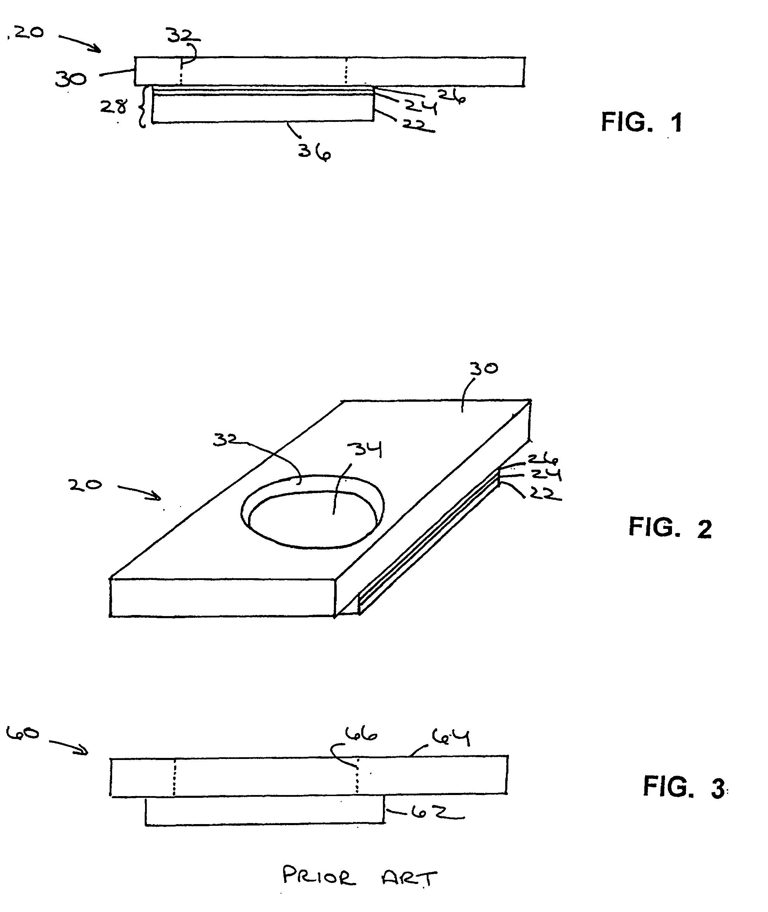

A first preferred embodiment of a test strip 20 according to the invention is illustrated in Figures 1 and 3. The test strip 20 includes a porous substrate 22 that is impregnated with a dried indicator formulation, the porous substrate 22 and its indicator formulation acting as a first layer of the structure. Overlying the porous substrate 22 and joined thereto is a second layer 24 that includes a dried reactive reagent formulation. Overlying the second layer 24 and joined thereto is a third layer 26 that includes a dried spreading agent formulation. The three layers 22, 24, and 26 are distinct, physically identifiable layers. There may be some slight intermixing between the layers due to the method of fabrication, to be described later, but generally the layers remain distinct.

The layers 22, 24, and 26 form an active unit 28, which is supported by and joined to a backing 30. The third layer 26 containing the spreading material is adjacent to the backing 30, and the porous substrate 22 containing the indicator formulation is remote from the backing 30. The layers 22, 24, and 26 and the backing 30 must be in this indicated order.

The backing 30 has an aperture 32 therethrough that registers with the layers 22, 24, and 26 of the unit 28. The aperture 32 is indicated with dotted lines in Figure 1, and is visible in the perspective view of Figure 2. The third layer 26 is visible through the aperture 32 from an upper side of the test strip 20. As may be seen in Figure 2, the aperture 32 in combination with the thickness of the backing 30 forms a well 34.

When the test strip 20 is used in its preferred application of glucose testing of blood, it is first placed into a reflective measurement apparatus of the type disclosed in US Patent 4,935,346, with the aperture 32 facing upwardly. A droplet of blood, whose glucose content is to be measured, is placed into the well 34 formed by the aperture 32. The spreading formulation of the third layer 26 is dissolved into the liquid, and aids in causing the droplet to spread over the entire surface of the third layer 26 that is exposed through the aperture 32.

The inventors have observed that, in the absence of the spreading formulation, the extent of the spreading of the droplet over the exposed surface of the third layer 26 varies with the size of the droplet that is placed into the well 34, the viscosity of the droplet, and the placement of the droplet. If a large droplet is placed into the well, the entire surface may be covered. If a smaller droplet is placed into the well or the placed droplet partially misses the well, only a portion of the surface of the apertured area may be covered. This variation in coverage leads to a variation in the final measured results of the test, because the optical reflectance instrument has a finite optical spot size on the porous substrate first layer 22 that may encompass both covered and uncovered regions.

In the present approach, by contrast, the spreading formulation aids in spreading small and/or misplaced droplets over the entire surface exposed through the aperture 32. Consequently, the test results are much less dependent upon the volume of the droplet than in the prior approach. This lower volume dependence of the test results is important, in view of the use of the test strip with the reflective measurement apparatus. These products are sold primarily for home diagnostic and measurement use by persons who have little or no scientific or medical training. It is difficult to ensure that these patients will always apply exactly the right amount of liquid in the test, and the present advance is important for these persons.

The presence of the spreading formulation also assists a person who may not deposit the droplet of blood precisely into the well 34. That is, many persons requiring glucose testing are infirm or elderly, and cannot easily draw a droplet of blood from themselves and place it precisely into the well. If the droplet is on the boundary of the well, the spreading layer spreads the droplet across the entire extent of the well.

After the droplet is placed into the well 34, the blood percolates downwardly through the second layer 24, where it dissolves the reagent formulation so that it may intermix with the liquid and the possible reactive substance therein. If the reactive substance is present, it reacts with the reagent formulation to form a reaction product. The reaction product mixes with the liquid of the droplet.

The droplet, with reaction products if any, percolates downwardly further into the porous substrate 22. In the porous substrate 22, the liquid dissolves indicator formulation into the droplet. If the reactive substance was originally present so that reaction product is present in the droplet at this point, the reaction product mixes with the dissolved indicator formulation and reacts therewith.

The reacted indicator formulation is detected by an appropriate technique. In the preferred approach, a light beam is directed against a back side 36 of the porous substrate 22. The light reflected from the back side 36 of the porous substrate 22 is analyzed to determine the presence of the reacted indicator formulation, which in turn is an indication of the presence and amount of the reactive substance in the original droplet. Complete details of the analytical instrument and the procedures are presented in US Patent 4,935,346. Further details of the chemical formulations of the various layers in the test strip 20 are presented subsequently.

In the discussion of the test strips 20, it has been emphasized that the indicator formulation is placed into one distinct layer, the porous substrate 22, and the reagent formulation is placed into a separate, distinct layer 24. This structure is to be contrasted with the structure of a prior test strip 60, shown in Figure 3. The test strip 60 has a porous substrate 62 joined to a backing 64 with an aperture 66 therethrough. Both an indicator formulation and a reagent formulation are impregnated into the porous substrate 62. The test strip 60 is used in substantially the same manner as the test strip 20.

Because the indicator formulation and the reagent formulation are mixed together within the porous substrate 62 of the test strip 60, they may partially react and degrade in performance during storage. Such a reaction is most likely in the presence of moisture. The manufacturer normally takes care to package the test strips inside a hermetically sealed container with a desiccant present to reduce moisture. Degradation of performance of the test strips during this sealed period occurs at a moderate rate. After the package is opened, the reaction between the indicator formulation and the reagent formulation is accelerated by the presence of moisture in the air or that otherwise may reach the test strips. Degradation is substantially accelerated after the container is opened.

In the present approach, by contrast, the indicator formulation and the reagent formulation are intentionally separated into two layers. Any degrading interaction occurs, if at all, more slowly than in the prior approach. The test strip 20 is to be marketed inside sealed containers with a desiccant, like the test strips 60. In the present approach, the multilayer test strip 20 degrades much more slowly than the single layer test strips 60, both before the packaging is opened and after it is opened. There is a finite shelf life and use life of the test strips 20, because the reactive formulation and the indicator formulation themselves can degrade without contact with the other, but the shelf life and use life are longer than with the single layer test strips 60.

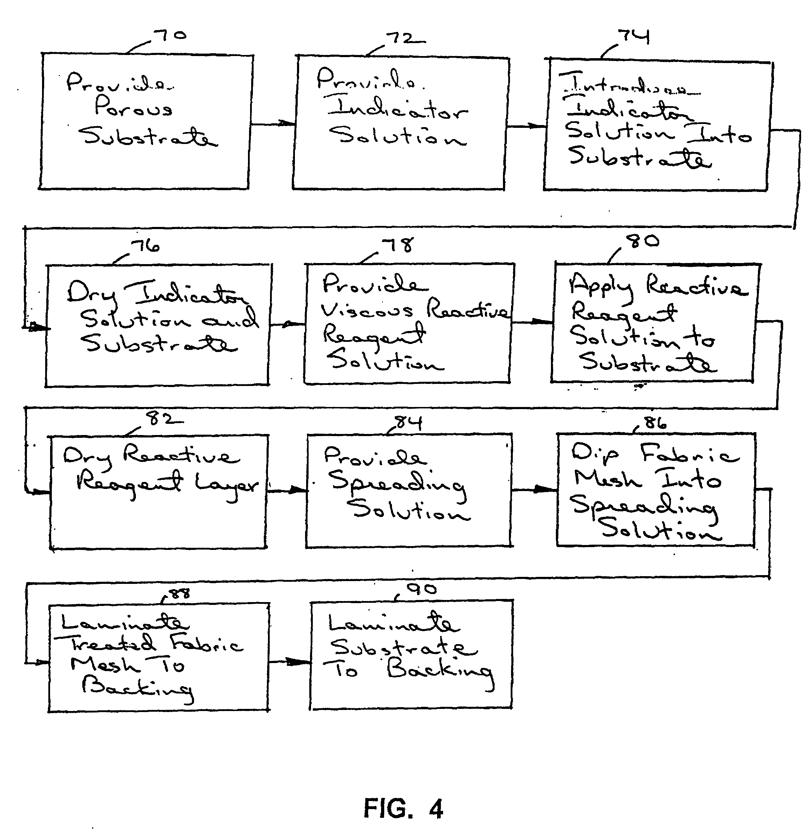

The procedure for preparing the test strip 20 of Figures 1 and 3 is depicted in Figure 4. A porous substrate 20 is first provided, numeral 70. The porous substrate 20 may be that described in the '346 patent at col. 4-6. In the preferred form of the present invention, the porous substrate is a piece of nylon membrane having a thickness of about 0,127 mm to 0,191 mm (0.005-0.0075 inches) and lateral dimensions of about 7,62 mm to 15,24 mm (0.3 by 0.6 inches). This porous substrate material is available commercially from Pall Corp. as Biodyne A. This material is made from nylon 66 and has a porosity of about 5 micrometers

A solution containing the indicator is provided, numeral 72. The preferred indicator is a dye whose appearance changes after a chemical reaction. A preferred indicator solution has a composition (for 100 milliliters of solution) of 1 gram DMAB (3-dimethylaminobenzoic acid), 30 milliliters of DMSO (dimethylsulfoxide), 0.33 grams Na₂HPO₄, 0.63 grams NaH₂PO₄, and 70 milliliters deionized water. The invention is not limited to this preferred indicator.

The indicator solution is introduced into the porous substrate 20, numeral 74. The above-described indicator solution is formulated to have a sufficiently low viscosity that it may be readily introduced by dipping the porous matrix into the solution for 30 seconds at ambient temperature, and permitting the excess solution to drain away.

The porous substrate 20 and its loading of indicator solution are dried, numeral 76, to remove the solvent from the porous substrate. Drying is preferably accomplished in air at 56C for 20 minutes. The indicator formulation is thereby dried and adhered to the fibers of the porous substrate.

A solution containing the reactive reagent formulation is provided, numeral 78. The preferred reactive reagent for glucose determination is a mixture of glucose oxidase and peroxidase. A preferred composition for the reactive reagent solution is 18000 I.U. glucose oxidase, 8040 I.U. peroxidase, 0.13 grams MBTH (3-methyl-2-benzothiazolinone hydrazone hydrochloride), 0.02 grams EDTA (ethylenediaminetetraacetic acid), 0.08 grams gelatin, 0.21 grams Na₂HPO₄, 0.46 grams NaH₂PO₄, 0.3 grams PVP (polyvinylpyrrolidone), 0.067 grams xanthan gum, 0.18 grams deoxycholic acid sodium salt, 0.1 grams DOBS (dodecylbenzene sulfonate sodium salt), and 26.4 milliliters distilled water.

The reactive reagent solution is applied as the separate layer 24 onto the porous substrate 20, numeral 80. The above-described reagent solution is prepared to have a sufficiently high viscosity that it may be readily coated as a layer onto the surface of the porous substrate 20, without penetrating into the porous substrate 20. The resulting layer 24 has a thickness of about 0,0254 mm (0.001 inch), when applied. Because the reactive reagent solution does have a liquid form, there is some interpenetration into the porous substrate 20, but that interpenetration is quite small. Such a slight degree of interpenetration is desirable, as it aids in adhering the layer 24 to the porous substrate 22 upon subsequent drying.

The reactive reagent layer is dried, numeral 82, to remove the solvent from the reactive reagent formulation. Drying is preferably accomplished in an oven at 60C. The reactive reagent formulation is thereby dried as the layer 24 which is adhered to the surface of the porous substrate 22.

A solution containing the spreading formulation is provided, numeral 84. The preferred spreading solution is a 0.2 percent solution of polyoxyethylene ether.

A piece of polyester fabric mesh is dipped into the spreading solution and dried, numeral 86. Drying is preferably accomplished in an oven at 60C.

The treated piece of fabric mesh is laminated to the backing 30, numeral 88. The backing is preferably a piece of polyvinylchloride having a thickness of about 0,127 mm to 0,254 mm (0.005-0.010 inches). An aperture having a diameter of about 5 millimeters is preformed through the backing. The treated piece of fabric mesh becomes the spreading layer or third layer 26 of the final test strip.

To complete the fabrication of the test strip, the laminate of backing 30 and third layer 26 is laminated to the previously prepared substrate 22 and second layer 24, numeral 90. In this laminate, the second layer 24 is adjacent to the third layer 26. During the processing procedures, there may be some slight interpenetration of the layers 24 and 26, but that interpenetration is quite small. Such a slight degree of interpenetration can be desirable, as it aids in adhering the layer 26 to the layer 24.

The pieces are usually prepared as large sheets, and diced into the appropriately sized individual test strips. The resulting structure is as shown in Figures 1 and 2.

Samples of the test strips have been prepared and tested to determine the glucose oxidase content of blood. Test strips prepared according to the present invention were tested both by themselves and comparatively with the prior test strips as shown in Figure 3. All measurements were performed using the "One Touch" optical measurement instrument manufactured by Lifescan, Inc., Milpitas, CA, generally in accordance with US patent 4,935,346, and its recommended procedure. The following results illustrate aspects of the invention, but should not be taken as limiting of the invention in any respect.

### Example 1--Hematocrit Dependence

Hematocrit is a measure of the blood viscosity of a person. The greater the hematocrit, the more viscous is the blood and the greater difficulty in causing the blood to spread across a surface. To test the significance of hematocrit in relation to the type of test strip, blood was prepared with an artificially introduced variation in hematocrit. Blood was diluted with plasma from the sample to produce artificial "blood" samples with hematocrits of 65%, 50%, 37%, and 27%. Two different glucose ranges were tested, a high range with a glucose of over 100 and a low range with a glucose of less than 100. The various blood hematocrit values (HCT) were tested for glucose content using the two types of test strips. Blood serum was prepared from the blood and tested for blood glucose content using the test strips to establish a measured 0 percent HCT value. The following table reports the results for the various blood hematocrit values that were tested.

| Test Strip | Measured Blood Glucose | | | | |
|---|---|---|---|---|---|
| HCT%: | 0 | 27 | 37 | 50 | 60 |
| Present Multilayer | | | | | |
| High Glucose | 329 | 314 | 309 | 278 | 245 |
| Low Glucose | 106 | 96 | 85 | 89 | 69 |

| Prior Single Layer | | | | | |
|---|---|---|---|---|---|
| High Glucose | 192 | 308 | 262 | 198 | 115 |
| Low Glucose | 78 | 89 | 82 | 62 | 50 |

Both test strips exhibit some dependence of the blood glucose content on blood HCT value. However, the multilayer test strip has a much lower dependence of its indicated blood glucose content on the hematocrit value than does the single layer test strip. The lesser dependence is particularly noticeable for the high blood glucose samples. The practical consequence of this result is that the present multilayer test strip gives results that are less dependent upon HCT value than does the prior single layer test strip.

## Claims

1. Test strip, comprising a dried indicator reagent and a dried reactive reagent which are jointly reactive with glucose to produce a measurable output in the event that glucose is present in a liquid sample, said test strip further comprising
- a first layer (22) comprising said indicator reagent impregnated into a porous substrate,
- a second layer (24) overlying the first layer (22) and comprising said reactive reagent,
- a third layer (26) contacting said second layer (24) and comprising a spreading agent promoting the spreading of a liquid upon said second layer (24) and
- an inert backing (30) overlying said third layer (26) and having an aperture (32) therethrough to said third layer (26), the aperture (32) being in registry with said first layer (22), said second layer (24) and said third layer (26),
wherein the first layer (22) incorporating the indicator reagent, the second layer including the reactive reagent and the third layer (26) including the dried spreading agent formulation are distinct from each other.

2. The test strip according to claim 1, wherein the porous substrate is a nylon membrane.

3. The test strip according to claim 1 or 2, wherein the indicator reagent is DMAB.

4. The test strip according to any of claims 1 to 3, wherein the reactive reagent is selected from the group consisting of glucose oxidase and peroxidase.

5. The test strip according to any of the preceding claims, wherein said third layer comprises a spreading agent that promotes the spreading of a droplet of blood through said third layer.

6. The test strip according to any of the preceding claims, wherein the spreading agent is selected from the group consisting of cellulose acetate and deatomaceous earth.

7. A method for preparing the test strip according to any of claims 1 to 6, said method comprising the steps of:
- providing a porous substrate,
- providing an indicator solution, the indicator solution having a sufficiently low viscosity that it is penetrable into said porous substrate,
- introducing said indicator solution into said porous substrate,
- drying said indicator solution within said porous substrate,
- providing a reactive solution containing a reagent, the solution having a sufficiently high viscosity that it is not penetrable into said porous substrate,
- applying said reactive reagent solution as a reactive reagent layer to a surface of said porous substrate,
- drying said reactive reagent within said reactive reagent layer,
- providing a spreading solution containing a spreading agent and
- applying said spreading solution as a spreading layer to the reactive reagent layer and
- attaching said porous substrate to a backing having an aperture therethrough with the applied layers adjacent to the backing and the porous substrate remote from the backing.

8. The method according to claim 7, wherein the indicator solution contains the indicator DMAB (2-dimethylaminobenzoic acid).

9. The method according to any of claims 7 or 8, wherein the reactive reagent solution contains a reagent selected from the group consisting of glucose oxidase and peroxidase.

## Patentansprüche

1. Testträgerstreifen, umfassend ein getrocknetes Indikatorreagens und ein getrocknetes reaktives Reagens, welche gemeinsam gegenüber Glucose reaktiv sind, um ein messbares Signal in dem Falle zu erzeugen, in dem in einer Flüssigprobe Glucose vorhanden ist, wobei der Testträgerstreifen des Weiteren umfasst:
- eine erste Schicht (22), die das Indikatorreagens in einer in ein poröses Substrat hineinimprägnierten Form umfasst,
- eine zweite Schicht (24), die über der ersten Schicht (22) liegt und das reaktive Reagens umfasst,
- eine dritte Schicht (26), die mit der zweiten Schicht (24) sich in Kontakt befindet und ein Ausbreitungsagens umfasst, das das Verlaufen einer Flüssigkeit auf der zweiten Schicht (24) beschleunigt sowie
- eine inerte Rückseite (30), die über der dritten Schicht (26) liegt und eine zur dritten Schicht (26) durchgehende Öffnung (32) aufweist, wobei die Öffnung (32) im Register mit der ersten Schicht (22), der zweiten Schicht (24) und der dritten Schicht (26) ist,
wobei die erste Schicht (22), die das Indikatorreagens inkorporiert aufweist, die zweite Schicht, die das reaktive Reagens beinhaltet und die dritte Schicht (26), die die getrocknete Formulierung mit dem Ausbreitungsagens einschließt, von einander verschieden sind.

2. Testträgerstreifen nach Anspruch 1, wobei es sich bei dem porösen Substrat um eine Nylon-Membran handelt.

3. Testträgerstreifen nach Anspruch 1 oder 2, wobei das Indikatorreagens DMAB ist.

4. Testträgerstreifen nach einem der Ansprüche 1 bis 3, wobei das reaktive Reagens ausgewählt ist aus der Gruppe, bestehend aus Glucose-Oxidase und Peroxidase.

5. Testträgerstreifen nach einem der vorhergehenden Ansprüche, wobei die dritte Schicht ein Ausbreitungsagens umfasst, das das Ausbreiten eines Tröpfchens Blut durch die dritte Schicht hindurch begünstigt.

6. Testträgerstreifen nach einem der vorhergehenden Ansprüche, wobei das Ausbreitungsagens ausgewählt ist aus der Gruppe, gestehend aus Celluloseacetat und Diatomeenerde.

7. Verfahren zum Herstellen des Testträgerstreifens nach einem der Ansprüche 1 bis 6, umfassend die Schritte, dass
- ein poröses Substrat bereitgestellt wird,
- eine Indikatorlösung bereitgestellt wird, die eine ausreichend niedrige Viskosität ausweist, so dass sie ausreichend in das poröse Substrat eindringt,
- die Indikatorlösung in das poröse Substrat eingebracht wird,
- die Indikatorlösung in dem porösen Substrat getrocknet wird,
- eine reaktive Lösung bereitgestellt wird, die ein Reagens enthält, wobei diese Lösung eine ausreichend hohe Viskosität aufweist, so dass sie nicht in das poröse Substrat eindringt,
- die Lösung mit dem reaktiven Reagens als eine Schicht mit einem reaktiven Reagens auf eine Oberfläche des porösen Substrats aufgebracht wird,
- das innerhalb der Schicht mit dem reaktiven Reagens sich befindende reaktive Reagens getrocknet wird,
- eine Ausbreitungslösung bereitgestellt wird, die ein Ausbreitungsagens enthält, und
- diese Ausbreitungslösung als eine Ausbreitungsschicht auf die Schicht mit dem reaktiven Reagens aufgebracht wird und
- das poröse Substrat an einer Rückseite anhaften gelassen wird, die eine Durchgangsöffnung aufweist, wobei die aufgebrachten Schichten der Rückseite benachbart sind und das poröse Substrat sich zurückgesetzt von der Rückseite befindet.

8. Verfahren nach Anspruch 7, wobei die Indikatorlösung den Indikator DMAB (2-Dimethylaminobenzoesäure) enthält.

9. Verfahren nach einem der Ansprüche 7 oder 8, wobei die Lösung mit dem reaktiven Reagens ein Reagens enthält, das ausgewählt ist aus der Gruppe, bestehend aus Glucoseoxidase und Peroxidase.

## Revendications

1. Bandelette-test comprenant un réactif indicateur séché et un réactif actif séché qui réagissent ensemble en présence de glucose pour produire un résultat mesurable dans le cas où du glucose est présent dans l'échantillon liquide, ladite bandelette-test comprenant en outre :
- une première couche (22) contenant ledit réactif indicateur imprégné dans un substrat poreux,
- une deuxième couche (24) recouvrant la première couche (22) et contenant ledit réactif actif,
- une troisième couche (26) en contact avec la deuxième couche (24) et contenant un agent de diffusion favorisant la diffusion du liquide sur ladite deuxième couche (24) et
- un support inerte (30) recouvrant ladite troisième couche (26) et comportant une ouverture (32) traversante jusqu'à ladite troisième couche (26), l'ouverture (32) étant alignée avec ladite première couche (22), ladite deuxième couche (24) et ladite troisième couche (26),
dans laquelle, la première couche (22) contenant le réactif indicateur, la deuxième couche comprenant le réactif actif et la troisième couche (26) comprenant l'agent de diffusion séché sont distinctes les unes des autres.

2. Bandelette-test selon la revendication 1, dans laquelle le substrat poreux est une membrane en nylon.

3. Bandelette-test selon la revendication 1 ou 2, dans laquelle le réactif indicateur est le DMAB.

4. Bandelette-test selon n'importe laquelle des revendications 1 à 3, dans laquelle le réactif est choisi parmi le groupe se composant de glucose-oxydase et de glucose-peroxydase.

5. Bandelette-test selon n'importe laquelle des revendications précédentes, dans laquelle ladite troisième couche contient un agent de diffusion qui favorise la diffusion d'une gouttelette de sang à travers ladite troisième couche.

6. Bandelette-test selon n'importe laquelle des revendications précédentes, dans laquelle l'agent de diffusion est choisi parmi le groupe se composant d'acétate de cellulose et de terre de diatomées.

7. Procédé pour préparer la bandelette-test selon n'importe laquelle des revendications 1 à 6, ledit procédé comprenant les étapes de:
- fourniture d'un substrat poreux,
- fourniture d'une solution indicateur, la solution indicateur ayant une viscosité suffisamment faible pour qu'elle puisse pénétrer dans ledit substrat poreux,
- introduction de ladite solution indicateur dans ledit substrat poreux,
- séchage de ladite solution indicateur à l'intérieur dudit substrat,
- fourniture d'une solution réactive contenant un réactif, la solution ayant une viscosité assez forte pour qu'elle ne puisse pas pénétrer dans ledit substrat poreux,
- application de ladite solution de réactif actif en une couche de réactif actif sur une surface dudit substrat poreux,
- séchage dudit réactif actif à l'intérieur de ladite couche de réactif actif,
- fourniture d'une solution de diffusion contenant un agent de diffusion et
- application de ladite solution de diffusion en une couche de diffusion sur la couche de réactif actif et
- liaison dudit substrat poreux à un support pourvu d'une ouverture traversante, les couches appliquées étant adjacentes au support et le substrat poreux étant distant du support.

8. Procédé selon la revendication 7, dans lequel la solution indicateur contient l'indicateur DMAB (acide 3-dimethylaminobenzoïque).

9. Procédé selon n'importe laquelle des revendications 7 ou 8, dans lequel la solution de réactif actif contient un réactif choisi parmi le groupe se composant de glucose-oxydase et de glucose-peroxydase.
